# EUROPEAN PATENT SPECIFICATION

(11) **EP 0 445 228 B1**
(45) Date of publication and mention of the grant of the patent: **20.01.1999**
(21) Application number: 90901248.6
(22) Date of filing: 22.11.1989
(51) Int. Cl.: A61K 39/395

(54) **IMMUNOTHERAPY INVOLVING CD28 STIMULATION**
IMMUNOTHERAPIE MIT EINBEZIEHUNG DER CD28-STIMULATION
IMMUNOTHERAPIE METTANT EN OEUVRE LA STIMULATION DE LA CD28

(30) Priority: 23.11.1988 US 275433
(43) Date of publication of application: 11.09.1991
(73) Proprietor: THE REGENTS OF THE UNIVERSITY OF MICHIGAN, Ann Arbor, Michigan 48109-1248 (US); Bristol-Myers Squibb Company, New York, N.Y. 10154 (US)
(72) Inventor: THOMPSON, Craig, B., Ann Arbor, MI 48104 (US); JUNE, Carl, H., Rockville, MD 20850 (US); LEDBETTER, Jeffrey, A., Seattle, WA 98177 (US); LINDSTEN, Tullia, Ann Arbor, MI 48104 (US)
(74) Representative: Woods, Geoffrey Corlett
(86) International application number: US8905304
(87) International publication number: WO9005541

(56) References cited:
- US-A- 4 654 210
- JOURNAL OF IMMUNOLOGY, vol. 136, no. 9, May 1983 (US); MARTIN, pp. 3282-3286/
- MOLECULAR & CELLULAR BIOLOGY, vol. 7, no. 12, December 1987 (US); JUNE, pp. 4472-4481/

## Description

The present invention generally relates to the use in the manufacture of a medicament of a ligand having binding specificity for at least a portion of the extracellular domain of the CD28 T cell surface molecule.

Thymus derived lymphocytes, referred to as T cells, are important regulators of in vivo immune responses. T cells are involved in cytotoxicity and delayed type hypersensitivity (DTH), and provide helper functions for B lymphocyte antibody production. In addition, T cells produce a variety of lymphokines which function as immunomodulatory molecules, such as for example, interleukin-2 (IL-2), which can facilitate the cell cycle progression of T cells; tumor necrosis factor-alpha (TNF-α) and lymphotoxin (LT), cytokines shown to be involved in the lysis of tumor cells; interferon-gamma (IFN-γ), which displays a wide variety of anti-viral and anti-tumor effects; and granulocyte-macrophage colony stimulating factor (GM-CSF), which functions as a multilineage hematopoietic factor.

Current immunotherapeutic treatments for diseases such as cancer, acquired immunodeficiency syndrome (AIDS) and attending infections, involve the systemic administration of lymphokines, such as IL-2 and IFN-γ, in an attempt to enhance the immune response by T cell proliferation. However, such treatment results in non-specific augmentation of the T cell-mediated immune response, since the lymphokines administered are not specifically directed against activated T cells proximate to the site of infection or the tumor. In addition, systemic infusions of these molecules in pharmacologic doses leads to significant toxicity. Present therapies for immunodeficient or immunodepressed patients also involve non-specific augmentation of the immune system using concentrated gamma globulin preparations or the systemic infusion of T cell lymphokines with disadvantageous systemic side effects. The stimulation of the in vivo secretion of immunomodulatory factors has not, until now, been considered a feasible alternative due to the failure to appreciate the effects and/or mechanism and attending benefits of such therapy.

It would thus be desirable to enhance the T-cell mediated immune response to direct it specifically toward T-cells activated by an antigen produced by the targeted cell. It would further be desirable to take advantage of the patient's natural immunospecificity. It would also be desirable to be able to secure these effects in immunodepressed patients. It would additionally be desirable to secure these effects by means which do not primarily rely on the administration of immunodulatory molecules in amounts having significant toxic effects.

It would also be desirable to avoid removal and reintroduction of T cell populations. It would further be desirable to provide a means not only of enhancing, but of suppressing T-cell mediated immunoresponses where such immunosuppression would be advantageous, for example, in transplant patients and in patients exhibiting shock syndrome.

### Summary of the Invention

The immunotherapeutic method of the present invention comprises the step of selectively regulating the in vivo level of a human T-cell lymphokine by administering a therapeutically effective amount of a ligand to a patient having a population of activated T cells, said ligand having binding specificity for at least a portion of the extracellular domain of the CD28 T-cell surface molecule.

The present invention takes advantage of the surprising and heretofore unappreciated effects of stimulation of the CD28 molecule of activated T cells. By activated T cells is meant cells in which the immune response has been initiated or "activated", generally by the interaction of the T cell receptor TCR/CD3 T cell surface complex with a foreign antigen or its equivalent. Such activation results in T cell proliferation and the induction of T cell effector functions such as lymphokine production. In this respect see prior art "Journal of Immunology", Vol. 136, No 9, May 1983, pp 3282 - 3286 and "Molecular and Cellular Biology", Vol. 7, No 12, December 1987, pp. 4472 - 4481.

Stimulation of the CD28 cell surface molecule with anti-CD28 antibody results in a marked increase of T cell proliferation and in IL-2 lymphokine levels when the T cell is activated by submaximal stimulation of its TCR/CD3 complex. Surprisingly, when the stimulation of the TCR/CD3 complex is maximized, upon co-stimulation with anti-CD28 there is a substantial increase in the levels of IL-2 lymphokine, although there is no significant increase in T cell proliferation over that induced by anti-CD3 alone. Even more surprisingly, not only are IL-2 levels significantly increased, but the levels of an entire set of lymphokines previously not associated with CD28 stimulation are increased. Remarkably, both the T cell proliferation and increased lymphokine production attributable to CD28 stimulation also exhibit resistance to immunosuppression by cyclosporine and glucocorticoids.

The present invention thus provides a means by which the T cell-mediated immune response can be regulated by stimulating the CD28 T cell surface molecule to aid the body in ridding itself of infection or cancer. The present invention can also be used not only to increase T cell proliferation, if so desired, but to augment the immune response by increasing the levels and production of an entire set of T cell lymphokines now known to be regulated by CD28 stimulation.

Moreover, because the effectiveness of CD28 stimulation in enhancing the T cell immune response appears to require T cell activation or some form of stimulation of the TCR/CD3 complex, the present invention can be used to selectively stimulate preactivated T cells capable of protecting the body against a particular infection or cancer, thereby avoiding the non-specific toxicities of the methods presently used to augment immune function. In addition, the present invention enhances T cell-mediated immune functions even under immunosuppressed conditions, thus being of particular benefit to individuals suffering from immunodeficiencies such as AIDS.

A better understanding of the present invention and its advantages will be had from a reading of the detailed description of the preferred embodiments taken in conjunction with the drawings and specific example set forth below.

### Brief Description of the Drawings

Figure 1 is a bar graph illustrating the absence of augmentation of the uptake of thymidine by CD28 stimulated T cells.
Figure 2 is a bar graph illustrating the increase in uridine incorporation by CD28 stimulation of anti-CD3 stimulated T cells.
Figure 3 is a graph illustrating the elevated cyclosporine resistance of T cell proliferation induced by CD28 stimulation.
Figure 4 is a Northern blot illustrating the effects of cyclosporine on PMA or anti-CD3 activated T cells lymphokine expression induced by anti-CD28.
Figure 5 is a graph of in vivo activation of T cells in monkeys by CD28 stimulation.

### Detailed Description of the Preferred Embodiments

In a preferred embodiment of the present invention, the CD28 molecule is stimulated to enhance the T cell-mediated immune response of antigen-activated T cells CD28 is a 44 kilodalton protein expressed on the surface of about 80% mature T cells which exhibits substantial homology to immunoglobulin genes. See Poggi, A., et al., Eur. J. Immunol., 17:1065-1068 (1987) and Aruffo, A., et al., PNAS (USA), 8573-8577 (1987). Binding of the CD28 molecule's extracellular domain with anti-CD28 antibodies in the present invention results in an increase in T cell proliferation and elevated lymphokine levels.

In Specific Examples III-IV and VI-VIII, T cell activation was accomplished by stimulating the T cell TCR/CD3 complex (which mediates the specificity of the T cell immune response) with immobilised anti-CD3 monoclonal antibodies, such as mAb G19-4, or by chemically stimulating with PMA and ionomycin. It should also be appreciated, however, that activation of the T cell can instead be accomplished by routes that do not directly involve CD3 stimulation, such as the stimulation of the CD2 surface protein.

In practice, however, an activated T cell population will be provided by the patient's own immune system, which, barring total immunosuppression, will have T cells activated in response to any foreign or substantially elevated level of antigen present due to disease or infection. The term "foreign antigen" is used broadly herein, meaning an antigen which is either not normally produced by the organism, or, as in carcinomas, an antigen which is not normally produced by the cell which is producing it. By "substantially elevated" level of antigen is meant an antigen level exceeding normal ranges and having potentially deleterious effects to the organism due to such elevation.

In accordance with the present invention, stimulation of the CD28 molecule itself is achieved by administration of a ligand, such as a monoclonal antibody or a portion thereof, having a binding specificity for CD28. Suitable antibodies include mAb 9.3, an IgG2a antibody which has been widely distributed and is available (for non-commercial purposes) upon request from Dr. Jeffrey A. Ledbetter of Oncogen Corporation, Seattle, WA, or mAb KOLT-2. Both these monoclonal antibodies have been shown to have binding specificity for the extracellular domain of CD28 as described in Leukocyte Typing II, Ch. 12, pgs. 147-156, ed. Reinhertz, E. L., et al. (1986). The F(ab')₂ fragment of mAb 9.3 is at present preferred, having been tested in vivo without adverse side effects reported. It should also be understood that the present invention contemplates the use of chimaeric antibodies as well as non-immunoglobulin ligands which bind the CD28 surface molecule.

The extracellular domain of CD28, which was sequenced by Aruffo, A., et al., PNAS (USA), 84:8573-8577 (1987), generally comprises the following amino acid sequence: 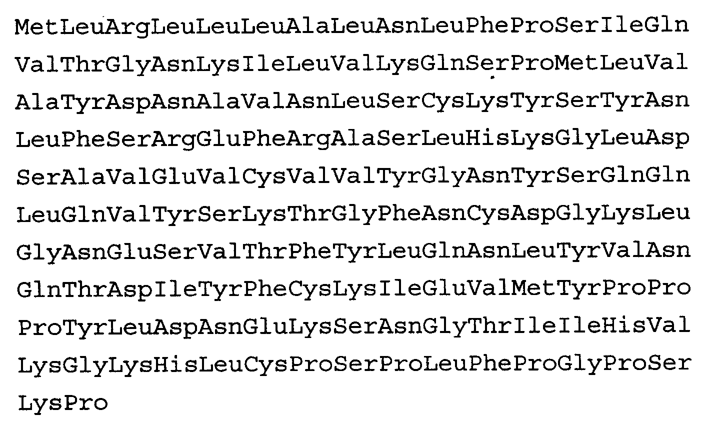 By the term "extracellular domain" as used hereinafter in the specification and claims, is meant the amino acid sequence set forth above, any substantial portion thereof, or any sequence having substantial homology thereto.

As shown by the data of Specific Examples III-V, substantial augmentation of the T cell-mediated immunoresponse by CD28 stimulation appears specific for activated T cells. Such specificity is of particular clinical importance and is one of the significant advantages of the present invention. Administration of anti-CD28 antibodies such as mAb 9.3 will specifically augment the response of T cells which are already activated and engaged in the immune response or those in the process of activation. It should, however, also be appreciated that CD28 stimulation may be effective even where the T cells are activated after the binding of the CD28 specific ligand of the present invention to CD28 receptor. Thus, the T cells at or near the tumor site or site of infection, which are being activated by the antigens produced or present at those sites, will be selectively "boosted" by the CD28 stimulation.

As previously discussed and further illustrated by the Specific Examples, the synergistic effect of CD28 stimulation on activated T cells results in increased T cell proliferation and increased IL-2 lymphokine levels when the TCR/CD3 complex is not maximally stimulated. However, when TCR/CD3 stimulation is maximised, although T cell proliferation is not markedly increased, the levels of certain lymphokines are substantially increased, indicating an increase in cellular production of these lymphokines. Thus, in patients undergoing natural maximal TCR/CD3 stimulation or its equivalent and T cell activation in vivo due to disease or infection, the administration of anti-CD28 antibody to stimulate CD28 in accordance with the method of the present invention will result in substantially elevated lymphokine production.

The increase in lymphokine production achieved by administration of CD28 stimulator in accordance with the method of the present invention, as particularly shown in Specific Example III, surprisingly results in the increased production of an entire set of lymphokines, indicating that these lymphokines are under some form of CD28 regulation. This set of lymphokines, which includes IL-2, TNF-α, LT, IFN-γ, and GM-CSF, is somewhat analogous to the T_{H}1 cell lymphokines present in the mouse which were described by Mosmann, T.R., et al., Immunol. Today, 8:223-227 (1987). Such finding is also buttressed by the lack of increase in human IL-4 production (data not shown) by CD28 stimulation, a lymphokine which is also not produced by the T_{H}1 cells of the mouse. Thus, for ease of reference, the group of human lymphokines affected by CD28 stimulation will hereinafter be referred to as human T_{H}1 lymphokines. It should be appreciated, however, that the term "human T_{H}1 lymphokines" is not limited to the lymphokines listed above, but is meant to include all human lymphokines whose production is affected or regulated by the binding or stimulation of the CD28 T cell surface molecule. Thus, by administration of anti-CD28 antibodies in accordance with the present invention, the production and levels of an entire set of human lymphokines can be significantly increased.

The present invention can also be used to facilitate the T cell-mediated immune response in immunodepressed patients, such as those suffering from AIDS. As shown in Specific Examples VI-VIII, T cell proliferation and the increased levels or production of CD28-regulated lymphokines continue to function even in the presence of immunosuppression such as that caused by cyclosporine or dexamethasone. Thus administration of CD28 stimulators such as mAb 9.3 can be used to treat immunodepressed patients to increase their in vivo lymphokine levels.

In addition, a variety of syndromes including septic shock and tumor-induced cachexia may involve activation of the CD28 pathway and augmented production of potentially toxic levels of lymphokines. Thus down-regulation of the CD28 pathway by, for example, binding CD28 with a F(ab')₂ fragment or a naturally occurring ligand for the CD28 molecule, can also be used for those clinical conditions.

It should be appreciated that the use of an anti-CD28 antibody has not heretofore been seriously contemplated as a potential immunotherapeutic method for the substantial increase of lymphokine levels at the sites of activated T cells. For example, the addition of mAb 9.3 has been thought only to somewhat augment T cell proliferation, not to induce substantial increases in human T_{H}1 lymphokine production.

Although it is not the intent herein to be bound by any particular mechanism by which CD28 binding regulates the T cell-mediated immune response, a model for the mechanism of stimulation has been postulated and supported with experimental data, some of which is shown in Specific Example VIII.

It has previously been shown that a number of lymphokine genes undergo more rapid degradation in the cytoplasm than mRNAs from constitutively expressed housekeeping genes, leading to the hypothesis that the instability of these inducible mRNAs has been selected to allow for rapid regulation of gene expression. It is believed that the mechanism of CD28 regulation herein described is related to the stabilization of rapidly degradable mRNAs for the set of human T_{H}1 lymphokines set forth above. To date, it appears no other mechanism in any eukararyotic cell system has been described to demonstrate that a cell surface activation pathway can alter gene expression by inducing specific alteration in mRNA degradation.

As shown in Specific Example IV, co-stimulation of CD28 and CD3 caused an increase in mRNA of the human T_{H}1 lymphokines which was not the result of a generalized increase in a steady state mRNA expression of all T cell activation-associated genes. The increase was disproportionate and thus could not be accounted for by the increase in percentage of proliferating cells in culture. These data, in addition to further studies not detailed herein, demonstrate that activation of the CD28 surface molecule of activated T cells functions to specifically stabilize lymphokine mRNAs. Increased mRNA stability, i.e. slower degradation thereof, results in increased translation of the mRNA, in turn resulting in increased lymphokine production per cell.

Thus, in accordance with the use of the present invention, ligands such as mAb 9.3 with binding specificity for the CD28 molecule are administered in a biologically compatible form suitable for administration in vivo to stimulate the CD28 pathway. By "stimulation of the CD28 pathway" is meant the stimulation of the CD28 molecule resulting in increased T cell proliferation or production of human T_{H}1 lymphokines or both. By "biologically compatible form suitable for administration in vivo" is meant a form of the ligand to be administered in which the toxic effects, if any, are outweighed by the therapeutic effects of the ligand. Administration of the CD28 ligand can be any suitable pharmacological form, which includes but is not limited to intravenous injection of the ligand in solution.

It should be understood that, although the models for CD28 regulation of lymphokine production are described with respect to stimulation and enhancement of lymphokine levels, down-regulation or inhibition of the CD28 pathway may also be possible by the selection of the appropriate ligand for CD28 binding: the ligand used then has an inhibitory effect on the CD28 pathway.

### SPECIFIC EXAMPLE I

### Preparation of CD28 Stimulator Monoclonal Antibody 9.3

The monoclonal antibody (mAb) 9.3, an IgG2a monoclonal antibody which binds to the extracellular domain of the CD28 molecule, was produced by a hybrid cell line originally derived by Hansen et al., as described in Immunogenetics, 10:247-260 (1980). Ascites fluid containing high titer monoclonal antibody 9.3 was prepared by intraperitoneal inoculation of 5-10 x 10⁶ hybrid cells into a Balb/C x C57BL/6 F₁ mice which had been primed intraperitoneally with 0.5 ml of Pristane (Aldrich Chemical Co., Milwaukee, WI). The monoclonal antibody 9.3 was purified from ascites fluid on a staphylococcal protein-A sepharose column as described by Hardy, R., Handbook of Experimental Immunology, Ch. 13 (1986). "Pristane" and "Sepharose" are Trade Marks.

Prior to use in functional assays, purified mAb 9.3 was dialyzed extensively against phosphate buffered saline (KCl 0.2 grams/liter dH₂O; KH₂PO₄ 0.2 grams/liter dH₂O; NaCl 8.0 grams/liter dH20; Na₂HPO₄·7H₂O 2.16 grams/liter dH₂O) and then filtered through a 0.22 cubic micron sterile filter (Acrodisc, Gelman Sciences, Ann Arbor, MI). The mAb 9.3 preparation was cleared of aggregates by centrifugation at 100,000 xg for 45 minutes at 20°C. The resulting purified mAb 9.3 was resuspended in phosphate buffered saline to a final concentration of 200µg/ml as determined by OD280 analysis and stored at 4°C prior to use.

### SPECIFIC EXAMPLE II

### Isolation of CD28⁺ T Cells

Buffy coats were obtained by leukopheresis of healthy donors 21 to 31 years of age. Peripheral blood lymphocytes (PBL), approximately 2.5 x 10⁹, were isolated from the buffy coat by Lymphocyte Separation Medium (Litton Bionetics, Kensington, MD) density gradient centrifugation. The CD28⁺ subset of T cells was then isolated from the PBL by negative selection using immunoabsorption, taking advantage of the reciprocal and non-overlapping distribution of the CD11 and CD28 surface antigens as described by Yamada et al., Eur. J. Immunol., 15:1164-1688 (1985). PBL were suspended at approximately 20 x 10⁶/ml in RPMI 1640 medium (GIBCO Laboratories, Grand Island, NY) containing 20mM HEPES buffer (pH 7.4) (GIBCO Laboratories, Grand Island, NY), 5mM EDTA (SIGMA Chemical Co., St. Louis, MO) and 5% heat-activated human AB serum (Pel-Freez, Brown Deer, WI). The cells were incubated at 4°C on a rotator with saturating amounts of monoclonal antibodies 60.1 (anti-CD11a) (see Bernstein, I.D., et al., Leukocyte Typing II, Vol. 3, pgs. 1-25, ed. Reinherz, E. L., et al., (1986); 1F5 (anti-CD20) (see Clark, E. A., et al., PNAS(USA), 82:1766-1770 (1985)); FC-2 (anti-CD16) (see June, C. H., et al., J. Clin. Invest., 77: 1224-1232 (1986)); and anti-CD14 for 20 minutes. This mixture of antibodies coated all B cells, monocytes, large granular lymphocytes and CD28⁻ T cells with mouse immunoglobulin. The cells were washed three times with PBS to remove unbound antibody, and then incubated for 1 hour at 4°C with goat anti-mouse immunoglobulin-coated magnetic particles (Dynal, Inc., Fort Lee, NJ) at a ratio of 3 magnetic particles per cell. Antibody-coated cells that were bound to magnetic particles were then removed by magnetic separation as described by Lea, T., et al., Scan. J. Immunol., 22:207-216 (1985). Typically, approximately 700 x 10⁶ CD28⁺ T cells were recovered.

Cell purification was routinely monitored by flow cytometry and histochemistry. Flow cytometry was performed as described by Ledbetter, J. A. et al., Lymphocyte Surface Antigens, p. 119-129 (ed. Heise, E., 1984). Briefly, CD28⁺ T cells were stained with fluorescein isothiocyanate (FITC)-conjugated anti-CD2 mAb OKT11 (Coulter, Hialeah, FL) and with FITC-conjugated anti-CD28 mAb 9.3 as described by Goding, J. W., Monoclonal Antibodies Principles and Practice, p. 230 (ed. Goding, J. W., 1983). CD28⁺ T cells were over 99% positive with FITC-conjugated monoclonal antibody OKT11 and over 98% positive FITC-conjugated monoclonal antibody 9.3 when compared to a non-binding, isotype-matched, FITC-labeled control antibody (Coulter, Hialeah, FL). Residual monocytes were quantitated by staining for non-specific esterase using a commercially available kit obtained from Sigma Chemical Co., St. Louis, MO and were less than 0.1% in all cell populations used in this study. Viability was approximately 98% as measured by trypan blue exclusion as described by Mishell, B.B., et al., Selected Methods Cell. Immunol., pgs. 16-17 (1980).

### SPECIFIC EXAMPLE III

### Increased Cellular Production of Human T_{H}1 Lymphokines by CD28 Stimulation by Monoclonal Antibody 9.3

CD28⁺ T cells were cultured at approximately 1 x 10⁵ cells/well in the presence of various combinations of stimulators. The stimulators included phorbol myristate acetate (PMA) (LC Services Corporation, Woburn, MA) at 3 ng/ml conc.; anti-CD28 mAb 9.3 at 100 ng/ml; anti-CD3 mAb G19-4 at 200 ng/ml which was immobilized by adsorbing to the surface of plastic tissue culture plates as previously described by Geppert, et al., J. Immunol., 138:1660-1666 (1987); also Ledbetter, et al, J. Immunol., 135: 2331-2336 (1985); ionomycin (Iono) (Molecular Probes, Calbiochem, CA) at 100 ng/ml. Culture supernatants were harvested at 24 hours and serial dilutions assayed for the human T_{H}1 lymphokines.

Specifically, IL-2 was assayed using a bioassay as previously described by Gillis et al., Nature, 268:154-156 (1977). One unit (U) was defined as the amount of IL-2 needed to induce half maximal proliferation of 7 x 10³ CTLL-2 (a human cytotoxic T cell line) cells at 24 hours of culture. In separate experiments the relative levels of IL-2 for each of the culture conditions above were independently confirmed using a commercially available ELISA assay (Genzyme Corp., Boston, MA). TNF-α/LT levels were measured using a semiautomated L929 fibroblast lytic assay as previously described by Kunkel et al., J. Biol. Chem., 263:5380-5384 (1988). Units of TNF-α/LT were defined using an internal standard for TNF-α (Genzyme Corp., Boston MA). The independent presence of both TNF-α and LT was confirmed by the ability of a monoclonal antibody specific for each cytokine to partially inhibit cell lysis mediated by the supernatant from cells co-stimulated with immobilized anti-CD3 mAb G19-4 and anti-CD28 mAb 9.3. IFN-γ was measured by radioimmunoassay using a commercially available kit (Centocor, Malvern, PA). Units for IFN-γ were determined from a standard curve using ¹²⁵I-labeled human IFN-γ provided in the test kit. GM-CSF was detected by stimulation of proliferation of the human GM-CSF-dependent cell line AML-193, as described by Lange et al., Blood, 70:192-199 (1987), in the presence of neutralizing monoclonal antibodies to TNF-α and LT. The ³H-thymidine uptake induced by 10 ng/ml of purified GM-CSF (Genetics Institute, Cambridge, MA) was defined as 100 U. Separate aliquots of cells were recovered 48 hours after stimulation and assayed for the percentage of cells in late stages of the cell cycle (S+G₂+M) by staining of cells with propidium iodide and analysis by flow cytometry as previously described by Thompson et al., Nature, 314:363-366 (1985).

As shown in Table 1, CD28 stimulation of CD3 stimulated T cells resulted in marked increases in cellular production of IL-2, TNF-α, IFN-γ and GM-CSF, herein referred to as human T_{H}1 lymphokines.

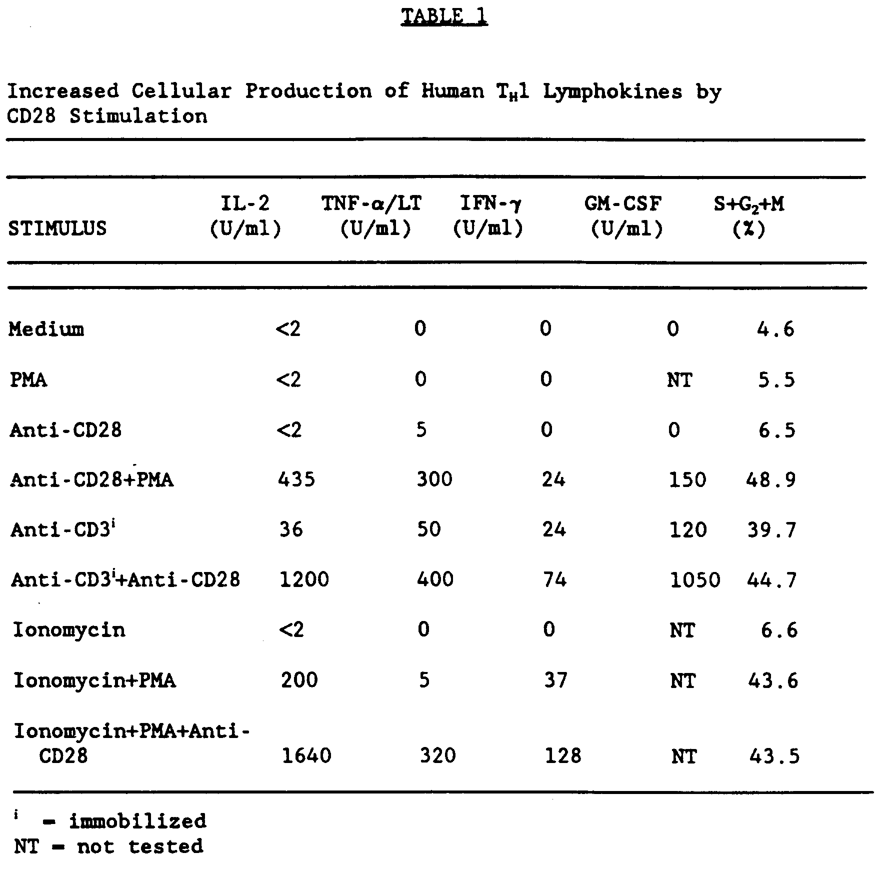

### SPECIFIC EXAMPLE IV

### Comparison of CD28 Stimulation to Stimulation of Other T Cell Surface Molecules

CD28⁺ T cells were cultured at approximately 1 x 10⁵ cells/well in RPMI media containing 5% heat-inactivated fetal calf serum (FCS), PHA 10 µg/ml, PMA 3 ng/ml, ionomycin at 100 ng/ml, anti-CD28 mAb 9.3 100 at ng/ml, or mAb 9.4 specific for CD45 at 1 µg/ml or mAb 9.6 specific for CD2 at 1 µg/ml, or immobilized mAb G19-4 specific for CD3 at 200 ng/well.

CD28⁺ T cells were cultured in quadruplicate samples in flat-bottomed 96-well microtiter plates in RPMI media containing 5% heat-inactivated fetal calf serum. Equal aliquots of cells were cultured for 18 hours and then pulsed for 6 hours with 1 uCi/well of ³H-uridine, or for 72 hours and then pulsed for 6 hours with 1 uCi/well of ³H-thymidine. The means and standard deviations (in cpm) were determined by liquid scintillation counting after cells were collected on glass fiber filters.

All cultures containing cells immobilized to plastic by anti-CD3 monoclonal antibodies were visually inspected to ensure complete cell harvesting. The failure of cells in these cultures to proliferate in response to PHA is the result of rigorous depletion of accessory cells, in vivo activated T cells, B cells, and CD11⁺ (CD28⁻) T cells by negative immunoabsorption as described in Specific Example II above. In each experiment, cells were stained with flourescein-conjugated anti-CD2 mAb OKT11 and flourescein-conjugated anti-CD28 mAb 9.3 and were shown to be over 99% and over 98% surface positive, respectively.

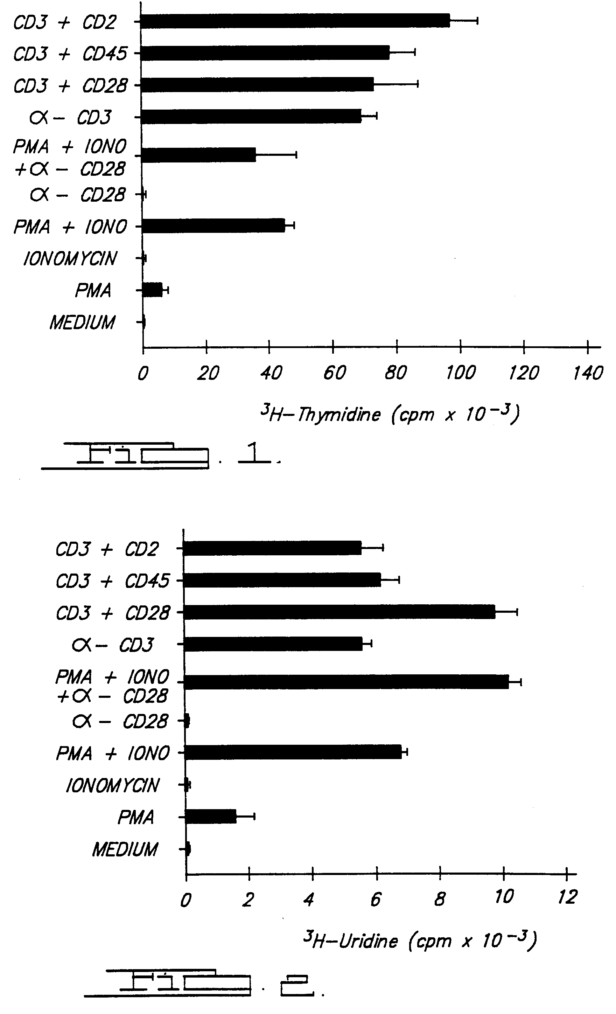

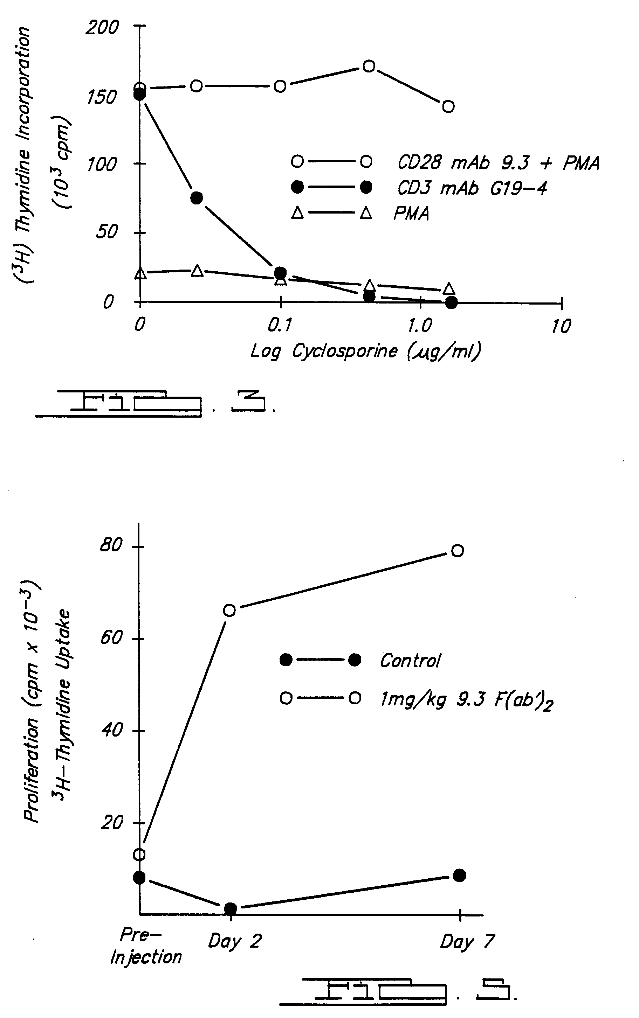

A representative experiment is illustrated in Figures 1 and 2. As shown in Figures 1 and 2, anti-CD28 by itself had no significant effect on uridine or thymidine incorporation, nor did it serve to augment proliferation induced either by immobilized anti-CD3 mAb G19-4 or chemically-induced T cell proliferation involving phorbol myristate acetate (PMA) and ionomycin (Iono). However, as shown in Figure 2, anti-CD28 did significantly increase the uridine incorporation of both sets of cells. In contrast, other monoclonal antibodies including anti-CD2 mAb OKT11 and anti-CD45 mAb 9.4 had no significant effect on uridine incorporation of anti-CD3 stimulated cells. This was not due to lack of effect of these antibodies on the cells, since both anti-CD2 and anti-CD7 monoclonal antibodies significantly augmented the proliferation of anti-CD3 stimulated cells. In separate experiments, the binding of isotype-matched mAbs to other T cell surface antigens (CD4, CD6, CD7 or CD8) and failed to mimic the effects observed with anti-CD28.

These data serve to confirm that the stimulation of activated T cells by CD28 has a unique phenotype which appears to directly enhance the rate of incorporation of a radioactive marker into the steady state RNA of T cells without directly enhancing T cell proliferation.

### SPECIFIC EXAMPLE V

### Increased Cellular Production of Human T_{H}1 Lymphokines by CD28 Stimulation Ex Vivo

Based on evidence from the in vitro systems it appeared that CD28 did not have a significant effect on cellular production of lymphokines unless they had undergone prior antigen activation or its equivalent. However, CD28 binding by the 9.3 mAb significantly enhanced the ability of anti-TCR/CD3 activated T cells to sustain production of human T_{H}1 type lymphokines. To test this effect in a physiologic setting, the activation of T lymphocytes in an ex vivo whole blood model was studied.

50-100 ml of venous blood was obtained by standard aseptic procedures from normal volunteers after obtaining informed consent. The blood was heparinized with 25 U/ml of preservative-free heparin (Spectrum, Gardenia, CA) to prevent clotting. Individual 10 ml aliquots were then placed on a rocking platform in a 15 ml polypropylene tube to maintain flow and aeration of the sample.

To assay for the effectiveness of CD28 stimulation on the induction of lymphokine gene expression, the production of TNF-α molecule was chosen as a model because of the extremely short half-life (approximately 15 minutes) of the protein in whole blood. 10 ml of whole blood isolated as described above was incubated with soluble anti-CD3 mAb G19-4 at a concentration of 1 µg/ml or anti-CD28 mAb 9.3 at a concentration of 1 µg/ml or a combination of the two antibodies. The plasma was assayed for TNF-α as described in Specific Example III at one and four hours. An example of one such experiment is shown in Table 1, which illustrates the significant increase in production of TNF-α by maximal stimulation of CD3 and co-stimulation of CD28.

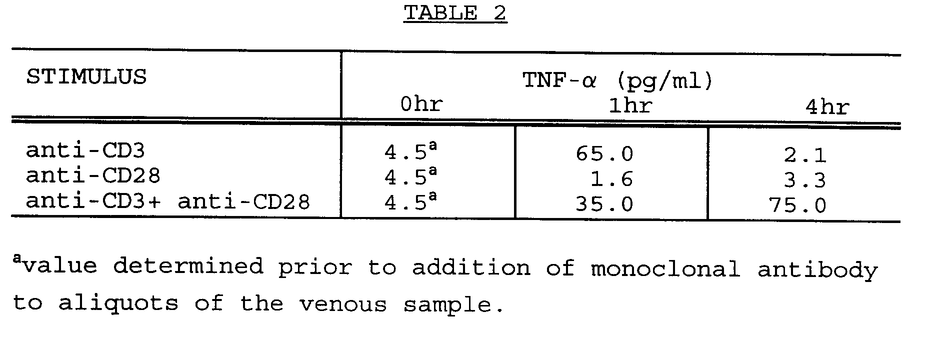

### SPECIFIC EXAMPLE VI

### Resistance of CD28-Induced T Cell Proliferation of Cyclosporine

The protocol used and results described herein are described in detail in June, C.H., et al., Mol. Cell. Biol., 7:4472-4481 (1987).

T cells, enriched by nylon wool filtration as described by Julius, et al., Euro. J. Immunol., 3:645-649 (1973), were cultured at approximately 5 x 10⁴/well in the presence of stimulators in the following combinations: anti-CD28 mAb 9.3 (100ng/ml) and PMA 1(ng/ml); or immobilized anti-CD3 mAb G19-4 (200ng/well); or PMA (100ng/ml). The above combinations also included fourfold titrations (from 25ng/ml to 1.6µg/ml) of cyclosporine (CSP) (Sandoz, Hanover, NJ) dissolved in ethanol-Tween 80 as described by Wiesinger, et al., Immunobiology, 156:454-463 (1979). "Tween" is a Trade Mark.

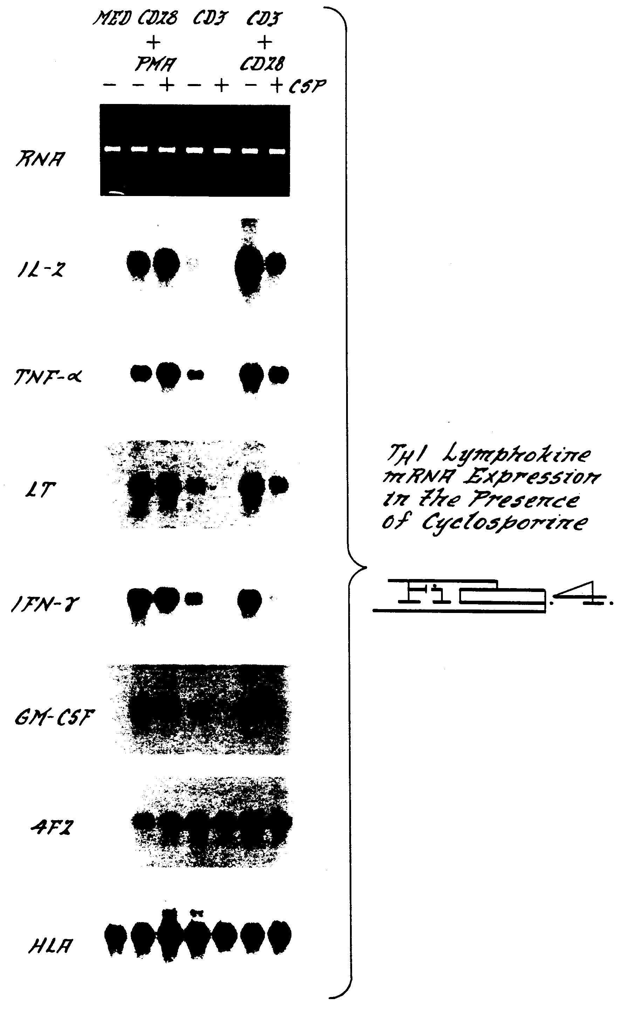

³H-thymidine incorporation was measured on day 3 of culture and the results representative of eight independent experiments are depicted in Figure 3. The arithmetic mean ± 1 standard deviation is depicted where the bar exceeds the size of the symbol. Proliferation of cells cultured in medium alone was 185 ± 40 cpm. The cyclosporine diluent alone did not affect cellular proliferation (data not shown). As shown in Figure 3, CD28-induced T cell proliferation exhibits nearly complete cyclosporine resistance when accompanied by the administration of PMA.

Table 3 below illustrates the effects of cyclosporine on CD3-induced proliferation of CD28⁺ T cells cultured at approximately 5 x 10⁴ cells/well in flat-bottomed 96-well microtites plates (CoStar, Cambridge, MA) under the following conditions: immobilized mAb G19-4; or immobilized mAb G19-4 and mAb 9.3 100ng/ml; or immobilized mAb G19-4 and PMA 1ng/ml; or mAb 9.3 100ng/ml and PMA 1ng/ml. Cyclosporine was prepared as above and included in the cultures at 0, 0.2, 0.4, 0.8, 1.2 µg/ml. ³H-thymidine incorporation was determined on day 3 of culture as above. The percent inhibition of proliferation was calculated between CD28⁺ T cells cultured in medium only or in cyclosporine at cyclosporine at 1.2 µg/ml. CD28⁺ T cells cultured in the absence of cyclosporine were given cyclosporine diluent. ³H-thymidine incorporation of cells cultured in medium, or PMA, or monoclonal antibody 9.3 only was less than 150 cpm. As shown in Table 3, co-stimulation of CD3 and CD28 resulted in a marked increase in the resistance of T cell proliferation to cyclosporine and the stimulation of CD28 in the presence of PMA resulted in a complete absence of cyclosporine suppression of T cell proliferation. Stimulation of CD28 together with immobilized anti-CD3 also resulted in resistance to suppression of T cell proliferation by the immuno-suppressant dexamethasone.

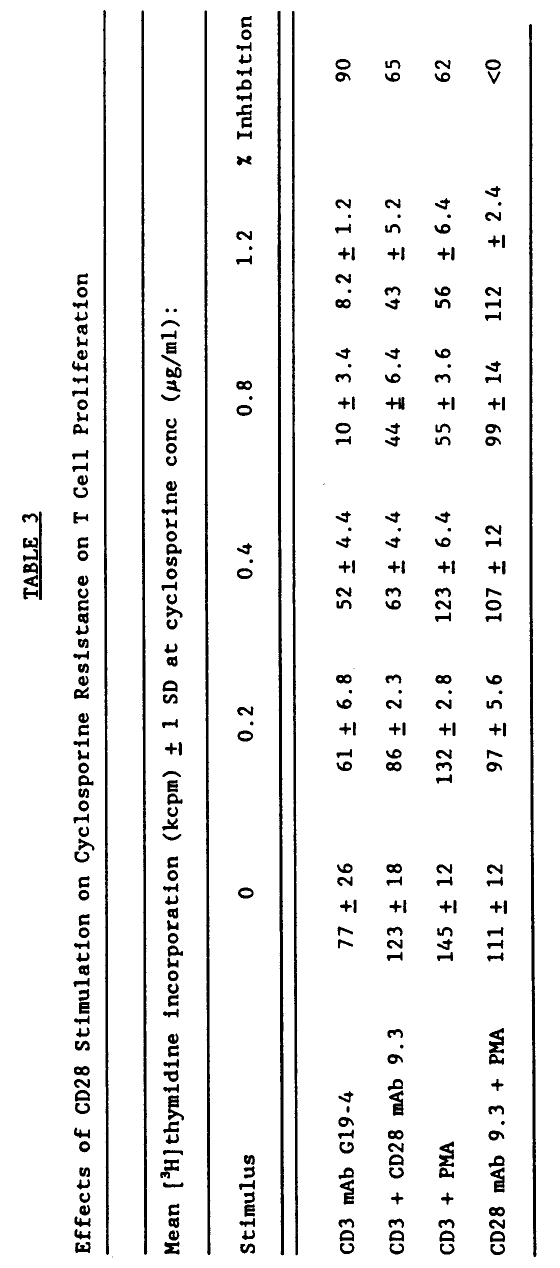

### SPECIFIC EXAMPLE VII

### Human T_{H}1 Lymphokine Secretion in the Presence of Cyclosporine

As described in Specific Example III, CD28⁺ T cells were cultured in the presence of various stimulators. Culture supernatants were harvested at 24 hours and serial dilutions assayed for IL-2, TNF-α/LT, IFN-γ, and GM-CSF as previously described. Separate aliquots of cells were recovered 48 hours after stimulation and assayed for the percentage of cells in late stages of the cell cycle (S+G₂+M).

When cyclosporine at 0.6 µg/ml was included in the test protocol, as shown in Table 4 (which also incorporates the data of Specific Example III for comparison), CD28⁺ T cells were found to secrete the human T_{H}1 lymphokines in the presence of cyclosporine in cultures stimulated with mAb 9.3 and PMA; or immobilized mAb G19-4 and mAb 9.3; or PMA and ionomycin and mAb 9.3. Human T_{H}1 lymphokine production induced by immobilized mAb G19-4; or by PMA with ionomycin was, however, completely suppressed in the presence of cyclosporine.

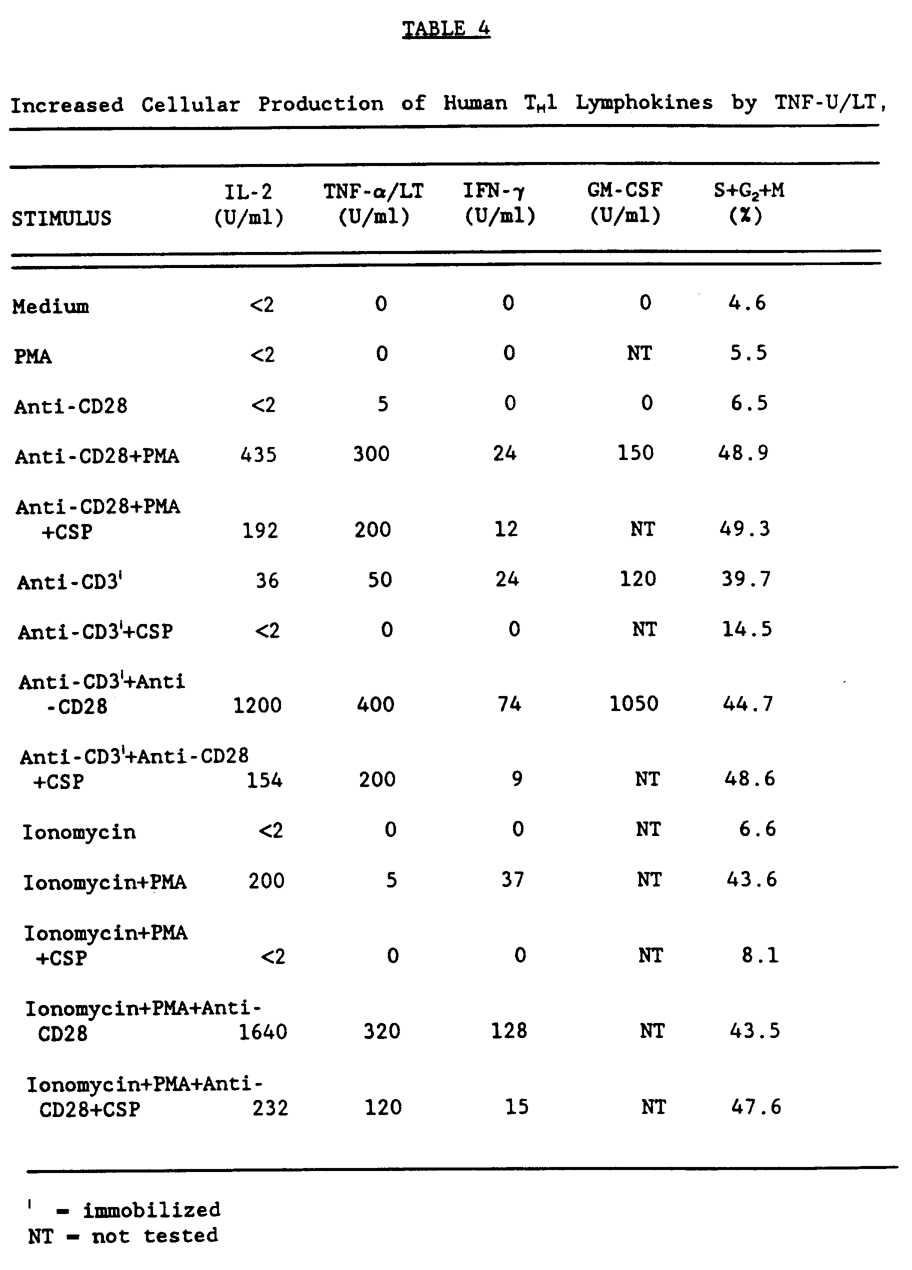

### SPECIFIC EXAMPLE VIII

### Human T_{H}1 Lymphokine mRNA Expression in the Presence of Cyclosporine

In order to further examine whether CD28 stimulation led to cyclosporine-resistant human T_{H}1 lymphokine gene expression as well as secretion, the ability of cyclosporine to suppress induction of IL-2, TNF-α, LT, IFN-γ, and GM-CSF following stimulation by various stimulators was tested. Specifically, CD28⁺ T cells were cultured at 2 x 10⁶/ml in complete RPM1 medium (GIBCO, Grand Island, NY) with 5% FCS (MED). Individual aliquots of CD28⁺ T cells were incubated for 6 hours in the presence or absence of 1.0 µg/ml cyclosporine with PMA 3ng/ml and anti-CD28 mAb 9.3 (1mg/ml); or with immobilized anti-CD3 mAb G19-4 (1 µg/well); or with immobilized mAb G19-4 (1 µg/well) and mAb 9.3 (1ng/ml). CD28⁺ T cells were harvested, total cellular RNA isolated and equalized for ribosomal RNA as previously described by Thompson, et al., Nature, 314:363-366 (1985).

Northern blots were prepared and hybridized sequentially with ³²P-labeled, nick-translated gene specific probes as described by June, C.H., et al., Mol. Cell. Biol., 7:4472-4481 (1987). The IL-2 probe was a 1.0 kb Pst I cDNA fragment as described by June, C.H., et al., Mol. Cell. Biol., 7:4472-4481 (1987); the IFN-γ probe was a 1.0 kb Pst I cDNA fragment as described by Young, et al., J. Immunol., 136:4700-4703 (1986). The GM-CSF probe was a 700 base pair EcoR I-Hind III cDNA fragment as described by Wong, et al., Science, 228:810-815 (1985); the 4F2 probe was a 1.85 kb EcoR I cDNA fragment as described by Lindsten, et al., Mol. Cell. Biol., 8:3820-3826 (1988); the IL4 probe was a 0.9 kb Xho I cDNA fragment as described by Yokota, et al., PNAS (USA), 83:5894-5898 (1986); and the human leukocyte antigen (HLA) probe was a 1.4 kb Pst I fragment from the HLA-B7 gene as described by Lindsten, et al., Mol. Cell. Biol., 8:3820-3826 (1988). TNF-α and LT specific probes were synthesized as gene specific 30 nucleotide oligomers as described by Steffen, et al., J. Immunol., 140:2621-2624 (1988) and Wang, et al., Science, 228:149-154 (1985). Following hybridization, blots were washed and exposed to autoradiography at -70°C. Quantitation of band densities was performed by densitometry as described in Lindsten, et al., Mol. Cell. Biol., 8:3820-3826 (1988).

As shown by the Northern blot of Figure 4, stimulation by mAb 9.3 with PMA and by mAb 9.3 with mAb G19-4 led to human T_{H}1 lymphokine gene expression that exhibited resistance to cyclosporine. In contrast, stimulation by mAb G19-4 alone was completely suppressed in the presence of cyclosporine.

### SPECIFIC EXAMPLE IX

### In Vivo Activation of T Cells by CD28 Stimulation

F(ab')₂ fragments of mAb 9.3 were prepared as described by Ledbetter, J. A., et al., J. Immunol., 135:2331-2336 (1985). Purified and endotoxin-free F(ab')₂ fragments were injected intravenously at 1 mg/kg of body weight over a 30 minute period into a healthy macaque (M. nemestrina) monkey. On days 2 and 7 after injection, 5 ml of blood was drawn and tested.

Peripheral blood lymphocytes from the monkey's blood were isolated by density grandient centrifugation as described in Specific Example II. Proliferation of peripheral blood mononuclear cells in response to PMA (1ng/ml) was tested in the treated monkey and a control animal (no F(ab')₂ fragment treatment) in triplicate as described in Specific Example IV. Proliferation was measured by the uptake of ³H-thymidine during the last 6 hours of a three-day experiment and the results shown in Figure 5. Means of triplicate culture are shown, and standard errors of the mean were less than 20% at each point. As shown in Figure 5, stimulation of CD28 by the F(ab')₂ mAb 9.3 fragment increased T cell proliferation in vivo.

## Claims

1. The use of a CD28 stimulatory ligand for the manufacture of a medicament for increasing the *in vivo* level of a human CD28 T-cell lymphokine produced by an activated CD28 T-cell in a patient having a population of CD28⁺ T-cells due to a disease or infection which results in maximal CD3 activation of the patient's CD28⁺ T-cells.

2. The use according to claim 1 wherein the CD28 stimulatory ligand is contacted *ex vivo* with a population of T-cells before introduction into a patient.

3. The use according to claim 1 or 2 wherein the human CD28 T-cell lymphokine is other than IL-2.

4. The use according to any one of the preceding claims wherein the CD28 stimulatory ligand comprises at least a portion of an anti-CD28 antibody.

5. The use according to claim 4 wherein the anti-CD28 antibody is characterised by the ability to induce the proliferation of cyclosporine and PMA treated CD28 T-cells *in vitro*.

6. The use according to claim 5 wherein an F(ab')₂ fragment of the antibody is used.

7. The use according to claim 4 wherein the anti-CD28 antibody is mAb 9.3.

8. The use according to any of claims 1 to 3 wherein the CD28 stimulatory ligand comprises a monoclonal antibody having the CD28 binding characteristics of Kolt-2.

## Patentansprüche

1. Verwendung eines CD28-stimulierenden Liganden für die Herstellung eines Medikaments zur Erhöhung des in vivo-Spiegels eines menschlichen CD28-T-Zell-Lymphokins, produziert von einer aktivierten CD28-T-Zelle in einem Patienten, der aufgrund einer Krankheit oder Infektion, die zu einer maximalen CD3-Aktivierung der CD28⁺-T-Zellen des Patienten führt, eine Population von CD28⁺-T-Zellen aufweist.

2. Verwendung nach Anspruch 1, wobei der CD28-stimulierende Ligand vor dem Einbringen in einen Patienten ex vivo mit einer Population von T-Zellen in Kontakt gebracht wird.

3. Verwendung nach Anspruch 1 oder 2, wobei das menschliche CD28-T-Zell-Lymphokin kein IL-2 ist.

4. Verwendung nach einem der vorangehenden Ansprüche, wobei der CD28-stimulierende Ligand mindestens einen Teil eines anti-CD28-Antikörpers umfaßt.

5. Verwendung nach Anspruch 4, wobei der anti-CD28-Antikörper gekennzeichnet ist durch die Fähigkeit, die Proliferation von Cyclosporin- und PMA-behandelten CD28-T-Zellen in vitro zu induzieren.

6. Verwendung nach Anspruch 5, wobei ein F(ab')₂-Fragment des Antikörpers verwendet wird.

7. Verwendung nach Anspruch 4, wobei der anti-CD28-Antikörper mAb 9.3 ist.

8. Verwendung nach einem der Ansprüche 1 bis 3, wobei der CD28-stimulierende Ligand einen monoclonalen Antikörper mit den CD28-Bindungseigenschaften von Kolt-2 umfaßt.

## Revendications

1. Emploi d'un ligand stimulant la CD28 pour fabriquer un médicament ayant pour effet d'augmenter le taux *in vivo* d'une lymphokine de cellules T CD28⁺ humaines, produite par des cellules T CD28⁺ activées, chez un patient possédant une population de cellules T CD28⁺, en raison d'une maladie ou d'une infection qui provoque une activation par CD3 maximale des cellules T CD28⁺ du patient.

2. Emploi conforme à la revendication 1, où le ligand stimulant la CD28 est mis en contact, *ex vivo*, avec une population de cellules T avant introduction chez un patient.

3. Emploi conforme à la revendication 1 ou 2, où la lymphokine de cellules T CD28⁺ humaines est autre que l'IL-2.

4. Emploi conforme à l'une des revendications précédentes, où le ligand stimulant la CD28 comporte au moins une partie d'un anti-corps anti-CD28.

5. Emploi conforme à la revendication 4, où l'anticorps anti-CD28 est caractérisé par sa capacité à provoquer *in vitro* la prolifération de cellules T CD28⁺ traitées à la cyclosporine et au PMA.

6. Emploi conforme à la revendication 5, où l'on utilise un fragment F(ab')₂ de l'anticorps.

7. Emploi conforme à la revendication 4, où l'anticorps anti-CD28 est l'anticorps mAb 9.3.

8. Emploi conforme à l'une des revendications 1 à 3, où le ligand stimulant la CD28 comporte un anticorps monoclonal présentant les caractéristiques de liaison à CD28 de l'anticorps Kolt-2.
